(19) Europäisches Patentamt
European Patent Office
Office européen des brevets

(11) **EP 4 722 717 A1**

(12) **EUROPEAN PATENT APPLICATION**

(43) Date of publication:
**08.04.2026 Bulletin 2026/15**

(21) Application number: **25205546.2**

(22) Date of filing: **30.09.2025**

(51) International Patent Classification (IPC):
***G01N 33/18*** (2006.01)   ***G01N 27/08*** (2006.01)

(52) Cooperative Patent Classification (CPC):
**G01N 33/1846; G01N 27/06; G01N 27/08**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **04.10.2024 US 202418907111**

(71) Applicant: **Mettler-Toledo Thornton, Inc.
Billerica MA 01821 (US)**

(72) Inventors:
• **Theriault, Robert
  Tyngsborough, MA 01821 (US)**
• **Reijnders, Anjan
  Groton, MA 01450 (US)**

(74) Representative: **Mettler-Toledo
IP Department
Im Langacher 44
8606 Greifensee (CH)**

(54) **DEVICES, SYSTEMS, AND METHODS FOR TOC ANALYSIS WITH AN ACTIVE FLOW**

(57)   Systems and methods for generating multiple data points for total organic carbon ("TOC") analysis of an actively flowing system are disclosed. A TOC device includes a sample fluid passageway and a flow control device, conductivity sensor(s), and an oxidation device located at the sample fluid passageway. A controller operates the flow control device to cause a flow of sample fluid through the sample fluid passageway at a first non-zero flow rate while the oxidation device is active, takes measurement(s) of the sample fluid from the conductivity sensor(s), operates the flow control device to cause a further flow of the sample fluid through the sample fluid passageway at a second non-zero flow rate while the oxidation device is active, and takes further measurement(s) of the sample fluid from the conductivity sensor(s), and generates delta conductivity measurements from the measurements.

FIG. 1

EP 4 722 717 A1

**Description**

**TECHNICAL FIELD**

Exemplary embodiments relate generally to devices, systems, and methods for TOC analysis with an active flow, such as by cyclically generating different flow rates of sample fluid to record oxidation curve data for total organic carbon ("TOC") analysis, which may include application of one or more artificial intelligence ("AI") models, such as to generate an error compensated TOC measurement and/or other findings.

**BACKGROUND AND SUMMARY OF THE INVENTION**

Various devices are known for analyzing TOC of fluids. Such devices may be important for measuring purity of water, which may be particularly useful for sensitive applications such as pharmaceutical applications, by way of non-limiting example. A certain level of sensitivity to particular compounds may be required to meet various regulatory or scientific standards (e.g., requirements). Some TOC sensor devices provide ongoing measurements for use with a continuously flowing system. Typically, such TOC sensor devices are fluidly connected to a larger fluid system and are configured to take (continuously, periodically, selectively, etc.) a sample of the flow in the larger fluid system for TOC analysis (hereinafter also "testing").

Some TOC sensor devices use conductivity sensors to measure conductivity of a sample fluid. Certain known approaches include taking a first conductivity measurement of the sample fluid, at least partially oxidizing the sample fluid, and taking a second conductivity measurement. At least in theory, the first measurement represents inorganic carbon (IC) in the sample fluid and the second measurement represents total carbon (TC). A difference in the conductivity measurements is used to derive a TOC measurement for the sample fluid, thereby representing the TOC measurement for the larger system. Scientific instruments, including TOC sensor devices, are subject to certain levels of error in measurement based on the unpredictability and randomness of various factors, such as flow rate of fluid measured, gas bubble interference within fluid, ultraviolet (UV) light exposure, temperature of fluid, and the like. Additionally, such measurements may be subject to a certain level of error based on the randomly occurring presence of interfering species in the sample fluid. Such interfering species include compounds within the sample fluid, at any point during the oxidation process, which are conductive (e.g., weak organic acids, strong inorganic acids, and conductive reactants) but do not necessarily oxidize completely to $CO_2$. These interfering species may be non-conductive compounds before oxidation which may chemically transform, generally through a number of intermediary conversions, into conductive compounds during the oxidation process. Examples of such conversions are provided below, by way of non-limiting example:

$$C_xH_yO_z + UV \text{ (e.g., 184 and 254nm)} \rightarrow CO_2 + H_2O \leftrightarrow H_2CO_3 \leftrightarrow H^+ + HCO_3^- \qquad \text{Example}$$
Conversion 1 (non-interfering)

$$CHCl_3 + UV \text{ (e.g., 184 and 254nm)} \rightarrow CO_2 + H_2O + HCl \leftrightarrow H_2CO_3 + HCl \leftrightarrow H^+ + Cl^- + HCO_3^-$$
Example Conversion 2 (interfering)

As such, these interfering species may be reflected in the second measurement, but not in the first measurement, thereby resulting in a too high TOC measurement.

Known approaches to minimizing error from the presence of interfering species generally involve separating carbon dioxide ($CO_2$) from the oxidized sample fluid (and thus also the interfering species). Such separation may be achieved by way of membrane separation (see e.g., US Pat. No. 5,132,094) or sparging (see e.g., US Pat. No. 9,791,430), for example, and the separated $CO_2$ may subsequently be measured with a sensor, such as a conductivity sensor, or a nondispersive infrared (NDIR) sensor, respectively. These approaches take time and are complicated to implement, increasing costs and necessary calibration efforts. Furthermore, taking such TOC measurements may take additional time, which may take time away from producing the desired product.

The inventive system and method for total organic carbon ("TOC") analysis with an active, such as continuous, flow device according to the claims overcomes these deficiencies.

The inventive method for generating multiple data points for total organic carbon ("TOC") analysis of an actively flowing system, said method comprising: operating a flow control device to cause a cyclic flow of a fluid through a sample fluid passageway between a plurality of non-zero flow rates while an oxidation device proximate the sample fluid passageway is active, and taking one or more measurements of the fluid from one or more conductivity sensors located at the sample fluid passageway at different times during the cycle; and
generating a plurality of delta conductivity measurements from the measurements.

The inventive system for generating multiple data points for total organic carbon ("TOC") analysis of an actively flowing system, said system comprising:

a TOC analysis device comprising:

a sample fluid passageway;
a flow control device located at the sample fluid passageway;
one or more conductivity sensors located at the sample fluid passageway; and
an oxidation device located at the sample fluid passageway; and

a controller comprising one or more non-transitory electronic storage devices comprising software instructions, which when executed, configure one or more processors to operate the flow control device to cause a flow of a fluid through the sample fluid passageway at a first non-zero flow rate while the oxidation device is active, and take one or more measurements of the fluid from the one or more conductivity sensors; operate the flow control device to cause a further flow of the fluid through the sample fluid passageway at a second non-zero flow rate while the oxidation device is active, and take one or more further measurements of the fluid from the one or more conductivity sensors; and generate a plurality of delta conductivity measurements from the measurements.

A further aspect of the present invention relates to a system according to claim 15.

Preferred embodiments of the aspects of the present invention are stated in the corresponding dependent claims and are described below.

These systems, and/or methods may be used to generate different non-zero flow rates of sample fluid, such as in a continuous, cyclic, and/or bimodal fashion, to record oxidation curve data for TOC analysis. Examples of continuous cyclical flow patterns provided by an active flow device include, but are not necessarily limited to, bimodal or square-wave, sawtooth, sinusoidal, and combinations thereof, or the like. The repetition of the cyclical flow patterns may or may not be of uniform time periods or frequencies. These devices, systems, and/or methods may include one or more artificial intelligence ("AI") models, such as to generate error compensated TOC measurements and/or other findings. The inventive solutions may provide enhanced accuracy without the need for $CO_2$ separation. TOC analysis may be provided in real time. Multiple data points of an oxidation curve may be gathered and/or generated while a sample flow is active. Curve fitting technique(s) may be used to derive a curve from the gathered data. Alternatively, or additionally, the gathered multiple data points may be used to generate an initial and/or an error compensated TOC measurement.

The inventive solutions may reduce errors and improve accuracy in measurement, such as, but not necessarily limited to, by way of reduced sensitivity to flow rate variability, UV lamp power output, temperature variability, gas bubble presence, background conductivity, combinations thereof, or the like, which may assist with meeting particular regulatory or scientific standards. Shorter warm up and/or measurement times, easier calibration, and/or shorter response times may be achieved. Furthermore, additional data insights may be provided, such as by way of the generated oxidation curve data. Examples of such additional insights include, but are not necessarily limited to, indication of type of interfering species encountered, self-improvement over time, error identification and/or classification, automated analysis, combinations thereof, or the like. By the gathered information, intelligent control over an associated system or components thereof may be provided.

A TOC analysis device may be fluidly connected to a fluid passageway with a larger flow. The TOC device may include one or more sample fluid passageways for a sample flow of sample fluid from the larger flow. The sample fluid passageway(s) may extend proximate to one or more oxidation devices, which may include one or more light sources, such as a UV lamp, to expose the fluid within the sample fluid passageway to the light (e.g., UV light) and cause oxidation of certain oxidation-capable compounds contained therein (herein after "oxidizing species"). For example, at least part of the sample fluid passageway may be arranged into a coil about a UV lamp. Conductivity sensors may be placed along the sample fluid passageway, such as at an entrance and exit of a coiled or other light-source-proximate portion of thereof.

To generate oxidation curves, the fluid may be provided through the sample fluid passageway at different flow rates over time, such as alternating between a relatively high and relatively low flow rate, thereby altering the residence/exposure time of various portions of the fluid, and therefore the relative oxidation levels of the oxidizing species contained therein. Conductivity measurements may be taken of the portions of the fluid experiencing the different residence/exposure times to generate multiple data points reflecting the oxidation curve of the sample fluid.

These multiple data points may be processed into regression curves, such as using one or more of various curve fitting techniques. Alternatively, or additionally, the multiple data points may be processed (e.g., conductivity measurements subtracted and resulting value compensated in various ways, such as for sample fluid temperature) to generate a TOC and/or ppbC measurement Alternatively, or additionally, the multiple data points may be processed through one or more AI models to provide an error compensated TOC and/or ppbC measurement, further understanding (e.g., classification of type of interfering species), insights, improve measurements over time, and/or provide control over an associated system or components thereof, such as by way of an artificial intelligence module.

In exemplary embodiments, without limitation, a system for generating multiple data points for TOC analysis of an actively flowing system is provided and includes, a TOC analysis device comprising: a sample fluid passageway, a flow control device located at the sample fluid passageway, one or more conductivity sensors located at the sample fluid passageway, and an oxidation device located at the sample fluid passageway. A controller includes one or more non-transitory electronic storage devices comprising software instructions, which when executed, configures one or more processors to: operate the flow control device to cause a flow of a fluid through the sample fluid passageway at a first non-zero flow rate while the oxidation device is active, and take one or more measurements of the fluid from the one or more conductivity sensors; operate the flow control device to cause a further flow of the fluid through the sample fluid passageway at a second non-zero flow rate while the oxidation device is active, and take one or more further measurements of the fluid from the one or more conductivity sensors; and generate a plurality of delta conductivity measurements from the measurements.

The controller may comprise additional software instructions stored at the one or more non-transitory electronic storage devices, which when executed, configures the one or more processors to: generate a regression curve from the plurality of delta conductivity measurements.

The oxidation device may comprise an ultraviolet ("UV") light source. The sample fluid passageway may be shaped, at least in part, into a coil about the light source. The one or more conductivity sensors comprises a first conductivity sensor positioned at an entrance to the coil and a second conductivity sensor positioned at an exit of the coil.

The flow control device may comprise at least one of: a pump, a valve, a gas pressure device, and a gravity feed device. The first non-zero flow rate may be higher than the second non-zero flow rate.

The flow of the fluid may have a first residence time within the sample fluid passageway, and the further flow of the fluid may have a second residence time within the sample fluid passageway.

The one or more measurements may comprise a first measurement taken while an entirety of the fluid in the sample fluid passageway is at the first non-zero flow rate. The one or more further measurements may comprise: a first further measurement taken while the fluid in the sample fluid passageway comprises a first portion at the first non-zero flow rate and a second portion at the second non-zero flow rate; and a second further measurement taken while an entirety of the fluid in the sample fluid passageway is, and has only been, at the second non-zero flow rate.

The controller may comprise additional software instructions stored at the one or more non-transitory electronic storage devices, which when executed, configures the one or more processors to: cycle between at least the first non-zero flow rate and the second non-zero flow rate.

The controller may comprise additional software instructions stored at the one or more non-transitory electronic storage devices, which when executed, configures the one or more processors to: continuously cycle between the first non-zero flow rate and the second non-zero flow rate in the bi-modal fashion.

The controller may be remote from the TOC analysis device.

The controller may comprise additional software instructions stored at the one or more non-transitory electronic storage devices, which when executed, configures the one or more processors to: determine a ppbC measurement from the plurality of delta conductivity measurements.

The controller may comprise additional software instructions stored at the one or more non-transitory electronic storage devices, which when executed, configures the one or more processors to: apply a deterministic model to the plurality of delta conductivity measurements to arrive at an initial TOC measurement.

The controller may comprise additional software instructions stored at the one or more non-transitory electronic storage devices, which when executed, configures the one or more processors to: apply an artificial intelligence module comprising a stochastic error model to the initial TOC measurement based on the plurality of delta conductivity measurements to arrive at an error compensated TOC measurement.

In exemplary embodiments, without limitation, a method for generating multiple data points for TOC analysis of an actively flowing system includes: operating a flow control device to cause a cyclic flow of a fluid through a sample fluid passageway between a plurality of non-zero flow rates while an oxidation device proximate the sample fluid passageway is active, and taking one or more measurements of the fluid from one or more conductivity sensors located at the sample fluid passageway at different times during the cycle; and generating a plurality of delta conductivity measurements from the measurements.

The method may include fitting, at a controller, a curve to the plurality of delta conductivity measurements.

The sample fluid passageway may be shaped, at least in part, into a coil about the light source. The light source may comprise an ultraviolet ("UV") lamp. The one or more conductivity sensors may comprise a first conductivity sensor positioned at an entrance to the coil and a second conductivity sensor positioned at an exit of the coil. The flow control device may comprise at least one of: a pump, a valve, a gas pressure device, and a gravity feed device.

The one or more measurements may comprise a first measurement taken while an entirety of the fluid in the sample fluid passageway is at the first flow rate. The one or more additional measurements comprise: a first additional measurement taken while the fluid in the sample fluid passageway comprises a first portion of the flow at the first flow rate and a second portion of the flow at the second flow rate, and a second additional measurement taken while an entirety of the fluid in the sample fluid passageway is at, and has been only at, the second flow rate.

The method may include applying, at the controller, a deterministic model to the plurality of delta conductivity measurements to arrive at an initial TOC measurement.

The method may include applying, at the controller, an artificial intelligence module comprising a stochastic error model to the initial TOC measurement based on the plurality of delta conductivity measurements to arrive at an error compensated TOC measurement.

The method may include determining, at the controller, a ppbC measurement from the error compensated TOC measurement.

In exemplary embodiments, without limitation, a system for generating multiple data points for TOC analysis of an actively flowing system comprises a TOC analysis device comprising: a sample fluid passageway for a fluid comprising a coiled portion, a flow control device located at an entrance or an exit to the coiled portion of the sample fluid passageway, said flow control device comprising at least one of: a pump, a valve, a gas pressure device, and a gravity feed device, a first conductivity sensor located at the entrance to the coiled portion of the sample fluid passageway, a second conductivity sensor located at the exit to the coiled portion of the sample fluid passageway, and an ultraviolet ("UV") lamp located within the coiled portion of the sample fluid passageway for irradiating the fluid within at least the coiled portion of the sample fluid passageway, when activated.

A controller includes one or more non-transitory electronic storage devices comprising software instructions, which when executed, configure one or more processors to: operate the flow control device to cause a flow of the fluid through the sample fluid passageway at a first flow rate while the UV lamp is active and irradiating the flow of the fluid; take a first conductivity measurement of the fluid from the first conductivity sensor and the second conductivity sensor while the coiled portion of the sample fluid passageway contains only the flow of the fluid at the first flow rate; operate the flow control device to cause a further flow of the fluid through the sample fluid passageway at a second flow rate while the UV lamp is active and irradiating the further flow of the fluid, where the second flow rate is lower than the first flow rate; take a second conductivity measurement of the fluid from the first conductivity sensor and the second conductivity sensor while the coiled portion of the sample fluid passageway contains the flow of the fluid at the first flow rate and at the second flow rate; take a third conductivity measurement of the fluid from the first conductivity sensor and the second conductivity sensor while the coiled portion of the sample fluid passageway contains only the flow of the fluid at the second flow rate; and generate a plurality of delta conductivity measurements from the first, second, and third conductivity measurements.

Further features and advantages of the systems and methods disclosed herein, as well as the structure and operation of various aspects of the present disclosure, are described in detail below with reference to the accompanying figures.

## BRIEF DESCRIPTION OF THE DRAWINGS

In addition to the features mentioned above, other aspects of the present invention will be readily apparent from the following descriptions of the drawings and exemplary embodiments, wherein like reference numerals across the several views refer to identical, similar, or equivalent features, and wherein:

FIGURE 1 is a schematic view of an exemplary active flow TOC analysis device and related system;

FIGURE 2 is a graph with an exemplary oxidation curve indicating exemplary measurement error possibilities;

FIGURE 3 is a graph with various exemplary oxidation curves;

FIGURE 4 is a flow chart with an exemplary method for operating the TOC analysis device of figure 1;

FIGURE 5 is a graph with an exemplary oxidation curve overlaid with multiple exemplary data points, such as gathered and/or processed using the TOC analysis device of figure 1 and/or method of figure 4;

FIGURE 6A is a graph demonstrating, in an exemplary fashion, how the TOC analysis device of figure 1 and/or method of figure 4 generate the oxidation curve data of figure 5 in an exemplary fashion;

FIGURE 6B is another graph further demonstrating, in an exemplary fashion, how the TOC analysis device of figure 1 and/or method of figure 4 generate the oxidation curve data of figure 5 in an exemplary fashion;

FIGURE 6C is another graph further demonstrating, in an exemplary fashion, how the TOC analysis device of figure 1 and/or method of figure 4 generate the oxidation curve data of figure 5 in an exemplary fashion;

FIGURE 7A is a schematic illustration of multiple data points that may be processed using the TOC analysis device of figure 1 and/or method of figure 4;

FIGURE 7B is an exemplary graphical representation of multiple data points from the measurements of figure 7A;

FIGURE 8 is a schematic overview and flow chart of an exemplary enhanced data processing method for multiple data points, such as obtained by the TOC analysis device of figure 1 and/or method of figure 4; and

FIGURE 9 is a schematic view of an exemplary TOC analysis system for the TOC analysis device of figure 1 and/or the methods of figures 4 and/or 8.

**DETAILED DESCRIPTION OF EXEMPLARY EMBODIMENT(S)**

Various embodiments of the present invention will now be described in detail with reference to the accompanying drawings. In the following description, specific details such as detailed configuration and components are merely provided to assist the overall understanding of these embodiments of the present invention. Therefore, it should be apparent to those skilled in the art that various changes and modifications of the embodiments described herein can be made without departing from the scope and spirit of the present invention. In addition, descriptions of well-known functions and constructions are omitted for clarity and conciseness.

Embodiments of the invention are described herein with reference to illustrations of idealized embodiments (and intermediate structures) of the invention. As such, variations from the shapes of the illustrations as a result, for example, of manufacturing techniques and/or tolerances, are to be expected. Thus, embodiments of the invention should not be construed as limited to the particular shapes of regions illustrated herein but are to include deviations in shapes that result, for example, from manufacturing.

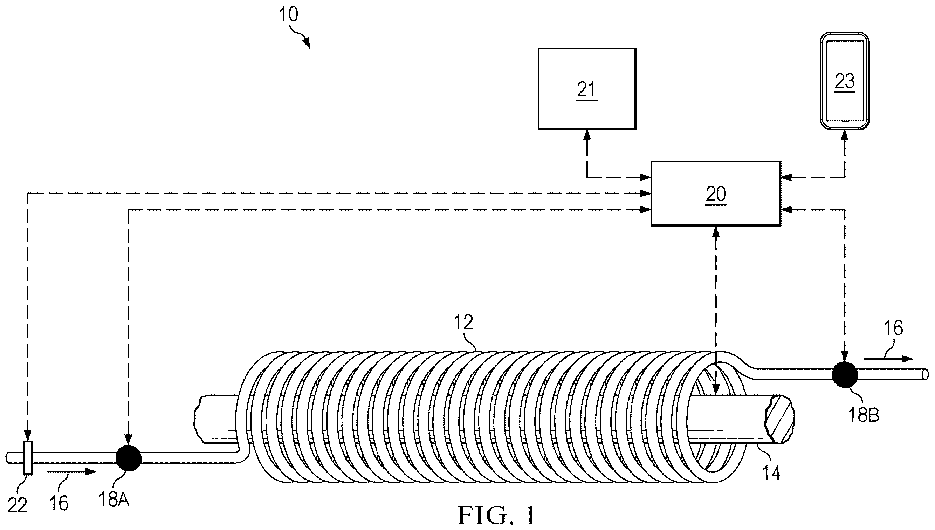

**FIGURE 1** illustrates an exemplary TOC device 10. The TOC device 10 may be an active flow analysis type device. The TOC device 10 may comprise a sample fluid passageway 12 and one or more oxidation devices, such as but not necessarily limited to, a light source 14, such as, but not necessarily limited to, an ultraviolet (UV) lamp. The light source 14 may be configured to, when activated, cause ultraviolet light at various wavelengths, such as at some wavelength between about 100nm and 400nm, such as 172nm, 185nm, and/or 254nm, by way of non-limiting example. When activated, the light source 14 may irradiate a flow of sample fluid 16 within the sample fluid passageway 12. The sample fluid passageway 12 may include any size or shape enclosed passageway (e.g., tube, pipe, duct, or the like) for a liquid and/or gas, such as purified water.

The light source 14 may be positioned sufficiently proximate to at least a first portion of the sample fluid passageway 12 to permit irradiation of the sample fluid 16 within at least the first portion of the sample fluid passageway 12 when the light source 14 is activated. In exemplary embodiments, without limitation, at least the first portion of the sample fluid passageway 12 may be provided in a coil or other shape about some or all of the light source 14, such as to keep the sample fluid 16 within a sufficient distance of the light source 14 to expose the sample fluid 16, thereby causing oxidation of any oxidizing species therein. Various size, shape, type, and/or number of sample fluid passageways 12 and/or light sources 14 may be utilized. At least the first portion of the sample fluid passageway 12 may comprise a UV transparent or translucent material, such as quartz by way of non-limiting example, to facilitate such oxidation from the light source 14.

Exposure of organic compounds in the sample fluid 16 to the UV light may result in oxidation, such as demonstrated by example conversion 1 and/or 2, without limitation.

Conductivity sensors 18 may be provided along the sample fluid passageway 12. In exemplary embodiments, without limitation, a first conductivity sensor 18A is provided at an entrance to a first portion of the sample fluid passageway 12 and a second conductivity sensor 18B is provided at an exit of the first portion of the sample fluid passageway 12. Other number, location, and/or type of such sensors 18 may be utilized within the sample fluid passageway 12. While a coil shape is sometimes shown and/or discussed, other arrangements and/or number of the sample fluid passageway 12 may be utilized, such as but not limited to, a shell and tube type arrangement (e.g., light(s) 14 surrounded by sample fluid passageway 12), light(s) 14 positioned outside and/or along the sample fluid passageway(s) 12, which may be straight, curved, in a zig-zag pattern, combinations thereof, or the like.

The TOC device 10 may comprise one or more flow control devices 22. The flow control device 22 may be positioned along the sample fluid passageway(s) 12 and may be electronically controlled or otherwise actuated to adjust a flow rate of the sample fluid 16 within the sample fluid passageway(s) 12. The flow control devices 22 may include valves, pumps, gas pressure devices, gravity feed devices, combinations thereof, or the like. The flow control device 22 may be positioned at an entrance to the first portion of the sample fluid passageway 12, for example. However, different number, type, and/or location of such flow control devices 22 may be utilized. For example, without limitation, the flow control devices 22 may be provided at the entrance and/or exit to the sample fluid passageway 12, along the sample fluid passageway 12, before or after the sample fluid passageway 12, combinations thereof, or the like.

The conductivity sensors 18, light source 14, flow control device 22, and/or other components of the TOC device 10 may be in electronic communication (wired and/or wireless) with a controller 20. The controller 20 may be part of the TOC device 10 or separate therefrom. The controller 20 may be configured to operationally control such components, such as by way of electronically issued commands to the same, and/or may be configured to receive multiple data points from the same, such as regarding operational status or data measurements.

In exemplary embodiments, without limitation, the controller 20 is configured to receive measurements from the sensors 18 while a flow of the sample fluid 16 through the sample fluid passageway 12 is active, and, preferably, while the light source 14 is also active. The measurements from the sensors 18 may be unconverted or may be converted to other units, or other formats, for further analysis and/or processing. A difference between the measurements from the first and second conductivity sensors 18A and 18B may be used by the controller 20 to determine a TOC measurement. For example, without limitation, the first conductivity sensor 18A may take a first conductivity measurement of the sample fluid 16 at the sample fluid passageway 12, which may be designated C1, and which may represent an inorganic carbon (IC) measurement of the sample. As the sample fluid 16 within the sample fluid passageway 12 is oxidized, such as by exposure, or continued exposure, to the light source 14, the second conductivity sensor 18B may take a second conductivity measurement, which may be designated C2, and which may represent a total carbon (TC) measurement of the sample fluid 16. The total organic carbon (TOC) may be calculated as TC - IC. In exemplary embodiments, without limitation, the TOC is calculated from a difference between the first and second conductivity measurements, which may be designated ΔC and/or referred to herein as a delta conductivity measurement, in accordance with the following equation:

$$TOC = function \ (\Delta C), \ where \ \Delta C = C2 - C1$$

## Equation 1

Each of the delta conductivity measurements (ΔC) may be processed to provide an initial TOC measurement, such as by way of one or more compensation adjustments (e.g., for sample fluid 16 temperature) and/or into a different form (e.g., ppbC). As further described herein, each of the delta conductivity measurements (ΔC), the first or second conductivity measurement of converted or unconverted units, and/or TOC measurements may be fit to a curve. As further described herein, these initial measurements may be error compensated, such as through various AI-utilizing techniques, to arrive at an error compensated TOC measurement, such as in ppbC.

Alternatively, or additionally, a single measurement from the second conductivity sensor 18B (which may be the only conductivity sensor 18 for the TOC device 10) may be used to generate some or all of the multiple data points, such as where the TOC device 10 is a batch processing device or an active flow device. In the case of an active flow device, an oxidation curve could be derived in certain situations from data from the conductivity sensor 18B only, by way of non-limiting example.

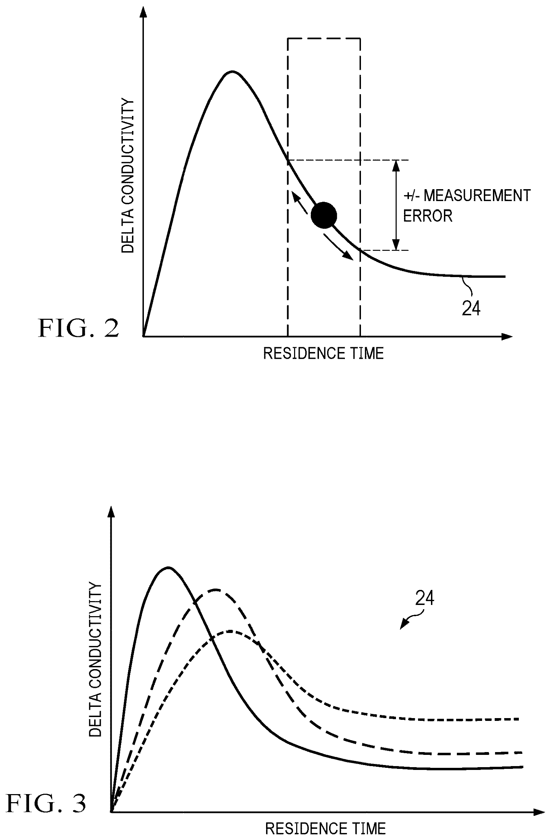

As demonstrated with particular regard to **FIGURE 2,** the delta conductivity measurement may vary with residence time of the sample fluid 16 within the first portion of the sample fluid passageway 12, commonly resulting in an oxidation curve 24 generally of a shape illustrated. This may be, for example without limitation, different species within the sample fluid 16 may oxidize into different chemical compounds over prolonged exposure, generally ultimately oxidizing into $CO_2$ if exposed for a sufficiently long period of time. Thus, as exposure/residence time varies, so will (generally) delta conductivity measurements. However, as further demonstrated with particular regard to **FIGURE 3,** various size and/or shape oxidation curves 24 may be generated, as such measurements are affected by various unpredictable and/or random factors, such as but not necessarily limited to, composition of the sample fluid 16, flow rate, gas bubble interference, UV dosage, temperature of the sample fluid 16, combinations thereof, or the like. Errors may persist even at complete oxidation, such as if all the reactant did not exclusively generate $CO_2$ and/or if the reactant was a conductive organic substance. The foregoing is not a comprehensive listing of error possibilities.

Longer residence times tend to result in more accurate measurements for at least certain interfering species. However, other interfering species can still cause errors further out on the oxidation curve. Regardless, longer residence times are undesirable as they interfere with production speed. Lower residence time may result in inaccurately high or low measurements, especially as the measurements shift due to variability in other factors (e.g., flow rate, gas bubble interference, UV dosage, temperature of fluid), examples of which are designated in figure 2, without limitation.

Traditionally, an oxidation curve for a particular sample could only be developed by batch processing and exposing a sample batch to some oxidizing condition over time (e.g., adding reagents, combustion with catalyst, UV exposure time, recycling oxidation loop, electrochemical oxidation, combinations thereof, or the like). For example, a sample fluid 16 may be provided within a container with a conductivity sensor and a UV light source proximate or within the container, and multiple data points are gathered as the sample is increasingly exposed to the UV light. Such approaches necessarily take additional time to gather the desired data. Traditionally, active (e.g., continuous) flow sensor device operate flow at, or substantially at (e.g., within 2% of), a given flow rate, resulting in a steady oxidation exposure/residence time and a resulting single data point measurement.

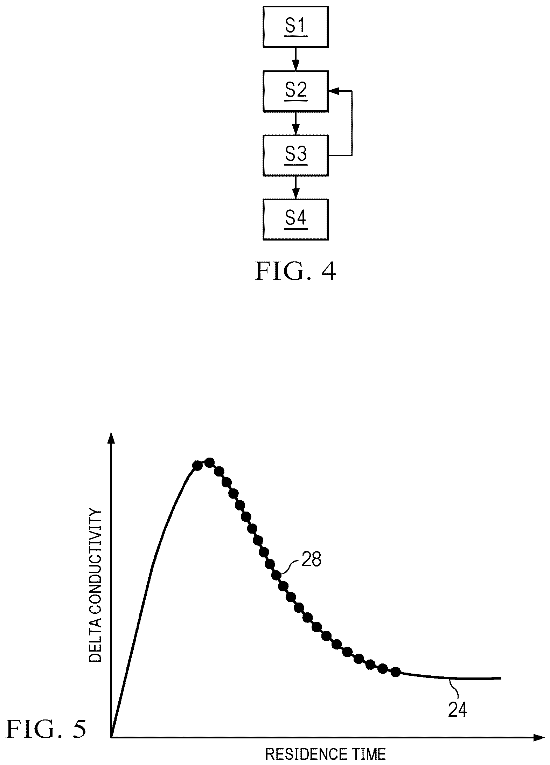

**FIGURE 4** illustrates a method for generating multiple data points for a flow of the sample fluid 16 at the TOC device 10, such as to generate an oxidation curve, such as while maintaining active (e.g., continuous) flow at the TOC device 10.

In exemplary embodiments, the following steps are performed: S1 - initiate sensor readings (ongoing), S2 - cause a high flow rate for a first period of time, S3 - cause a low flow rate for a second period of time, and S4 generate a TOC analysis from sensor readings. Steps S2 and S3 may be repeated, such as in a cyclical fashion.

The flow of the sample fluid 16 through the sample fluid passageway 12, or at least the first portion thereof, may be provided at different flow rates over time. In exemplary embodiments, without limitation, the sample fluid 16 is provided at a relatively high flow rate for a first period of time. For example, without limitation, the sample fluid 16 may be provided at a flow rate between 10 and 100 ml/min for a time period between 1 and 125 seconds. The flow may subsequently be switched to a relatively low flow rate for a second period of time. For example, without limitation, the sample fluid 16 may be provided at a rate of between 1 and 30 ml/min for a time period between 1 and 125 seconds. The first and second periods of time may be the same or different. The flow rates and times shown and/or described herein are exemplary and not intended to be limiting. In exemplary embodiments, without limitation, the flow rates and times may be adjusted based on various characteristics of the TOC device 10 (e.g., sample fluid passageway 12 size and/or shape) such as to provide a variety of residence times of the sample fluid 16 within the sample fluid passageway 12 so as to generate the desired data. The relatively low flow rate may provide the sample fluid 16 with a first residence time within the sample fluid passageway 12, while the relatively high flow rate may provide the sample fluid 16 with a second residence time within the sample fluid passageway 12, which is shorter than the first residence time. Alternatively, or additionally, a volume of the sample fluid 16 at the high flow rate may be provided through the TOC device 10 which at least equals, or is up to 5 times, preferably about three times, a volume of the sample fluid passageway 12 of the TOC device 10. In exemplary embodiments, without limitation, the high and low flow rates will continue in a cyclical, preferably continuously cyclical, fashion by a bimodal, square-wave pattern, combinations thereof, or the like.

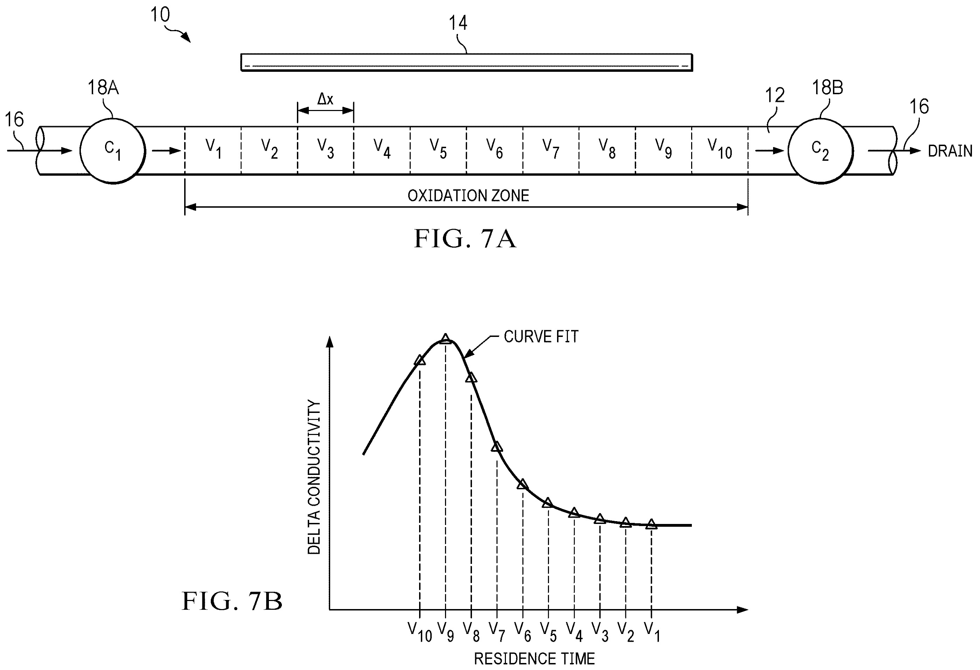

As illustrated with particular regard to **FIGURE 7A** and **FIGURE 7B,** and further discussed herein, at one or more times while the different flow rates are underway, measurements may be taken by the sensors 18. With particular regard to **FIGURE 7A,** as the sample fluid 16 passes from an intake or entrance through the sample fluid passageway 12, at least a portion of which is exposed to the light source 14 (hereinafter sometimes also called the "oxidation zone"), the sample fluid 16 may be exposed to various levels of oxidation over time before exiting the sample fluid passageway 12 by way of a drain or exit. Sensor 18 data may be taken at continuous intervals while the TOC device 10 is operational (e.g., while the flow rate is changed), only before and/or after flow changes, or the like. "V" (i.e., $V_1$, $V_2$, $V_3$, etc.) may represent discrete volumes of the sample fluid 16, each of which may have a different value when measured by the sensor(s) 18, where "x" may represent the size of the volume of the sample fluid 16 and/or the sample fluid passageway 12 containing the sample fluid 16 under consideration. After switching from the high flow to the low flow, the first portion of the sample fluid 16 within the sample fluid passageway 12 that is measured by the sensor 18B will yield a $\Delta C$ value corresponding to "V10", by way of example. This $\Delta C$ value will also correspond to a unique residence time reflective of the high flow. Subsequently each additional portion of fluid as indicated by "V9" , "V8", "V7", etc. will correspond to increasing unique residence times reflective of a combination of the high and low flows along with the corresponding $\Delta C$ values. As $\Delta x$ decreases to the size of a differential element, the conductivity sensor 18B is able to measure an increasingly larger number of differential sample fluid volumes (dV) with each differential sample fluid volume corresponding to a unique ordered pair comprised of a residence time and a corresponding $\Delta C$ value. The number of ordered pairs generated in the TOC device 10 represents a unique oxidation curve for the sample fluid 16 within the sample fluid passageway 12. The number of discrete data points representing the oxidation curve in further analysis may be determined by the data log settings located in the controller 20. With particular regard to **FIGURE 7B,** the multiple data points from the sensors 18 (e.g., $V_1$, $V_2$, $V_3$, etc.) may be curve fit, such that the multiple data points could be graphically represented in the form of $\Delta C$ values ($C_2 - C_1$) on the Y-axis against residence time of the sample fluid 16 on the x-axis.

In exemplary embodiments, without limitation, at least one measurement is taken while at least the first portion of the sample fluid passageway 12 is filled with only the relatively high flow rate sample fluid 16, at least one other measurement is taken while at least the first portion of the sample fluid passageway 12 is filled with a combination of high and low flow rate sample fluid 16 (e.g., the high flow rate sample fluid 16 is being displaced by the low flow rate sample fluid 16), and at least one other measurement is taken while at least the first portion of the sample fluid passageway 12 is filled with only the relatively low flow rate sample fluid 16. However, a large variety of measurements may be taken at various times, such as to reflect various residence times of the sample fluid 16.

Alternatively, or additionally, the low flow rate may be first introduced with the high flow rate after. In this fashion, the TOC device 10 may operate with a cyclic and/or bimodal flow, which is an exemplary embodiment. Alternatively, or additionally, a larger number of different flow rates may be provided (e.g., high, medium, and low, four different flow rates, etc.), such as in a cyclical, preferably continuously cyclical, fashion. The flow rates may be provided as step-changes, gradual changes, sinusoidal changes, saw tooth changes, combinations thereof, or the like. Regardless, alternating and/or changing between the different flow rates may be repeated, in a same or different order.

In yet other exemplary embodiments, without limitation, a plurality of conductivity sensors 18 may be posited along the sample fluid passageway 12 which may have a constant flow rate of the sample fluid 16 over time. This may provide a plurality of conductivity measurements reflecting various residence and/or exposure times of the sample fluid 16 to the light source 14.

Regardless of how the multiple data points are generated, the multiple data points may be taken of portion(s) of the sample fluid 16 having different residence times in the sample fluid passageway 12 (and therefore different UV exposure/oxidation levels from the light source 14). The multiple data points may be collectively formed into a regression curve shape and/or a curve reflective of a complete oxidation curve, or part of an oxidation curve, such as illustrated with particular regard to **FIGURE 5** at item 28. In this way, the multiple data points may be reflective of the oxidation curve of the sample fluid 16, as generated by the TOC device 10.

**EP 4 722 717 A1**

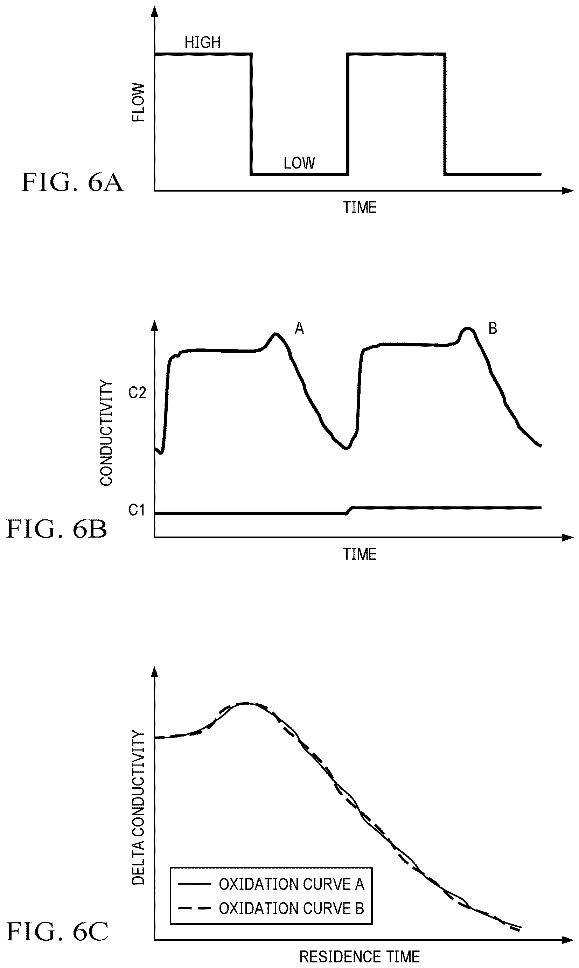

Examples of such data measurements and TOC device 10 operations are illustrated with regard to **FIGURE 6A** through **FIGURE 6C** by way of non-limiting example. As illustrated generally at **FIGURE 6A,** the TOC device 10 may be configured to automatically (e.g., in a pre-programmed or otherwise controlled fashion) switch between two (or more) different flow rates (e.g., high, low) over time, such as to provide a multimodal (e.g., bimodal) cycle of different flow rates. As illustrated generally at **FIGURE 6B** this may result in a continuous oxidation curve as the oxidizing species within the sample fluid 16 oxidizes over time based on exposure and/or residence time, such changes being represented by discrete conductivity measurements by the sensors 18. Those measurements may be transformed into delta conductivity measurements, such as at the controller 20, as illustrated generally at **FIGURE 6C.** Actual measurements/ multiple data points may represent discrete gathered conductivity measurements over time. A curve may be fit to those discrete multiple data points, or such multiple data points may be fit to a curve. Examples of such data measurements are illustrated with regard to **FIGURE 7A** and/or **FIGURE 7B,** without limitation.

These steps (e.g., switching between relatively high and low flow rates) may be repeated to generate additional curves and/or gather additional data points over a continuous flow of the sample fluid 16. For instance, a continuous, active flow of the sample fluid 16 may be passed through the TOC device 10 at the different flow rates to generate the multiple data points for the (complete or partial) oxidation curve. In this regard, an assumption may be made that the sample fluid 16 is the same, or substantially the same (e.g., in composition, temperature, etc.), over the time measured.

Other techniques for altering residence/exposure times may be utilized. For example, without limitation, the sample fluid 16 may be recycled through the TOC device 10 (at a same or different flow rate) to differentially expose the sample fluid 16 to ultraviolet light from the light source 14. As another example, without limitation, an electrochemical oxidation device may be utilized at a constant flow. Conductivity measurements may be taken by sensor 18 located at, or proximate, the inlet and outlet of the electrochemical oxidation device. By varying operating parameters of the electrochemical oxidation device (e.g., current, voltage, etc.) differing oxidation conditions are generated.

In exemplary embodiments, without limitation, the different flow rates (e.g., switching between high and low flow rates) may be selectively initiated, such as automatically at certain times, periodically, manually, in response to certain conditions, combinations thereof, or the like. For example, without limitation, the TOC device 10 may be configured to automatically initiate the different flows and subsequent operations (e.g., gather data, process results) upon start-up of the larger system, regularly during operation, randomly, combinations thereof, or the like. As another example, without limitation, the TOC device 10 may be configured to normally operate at a baseline flow (e.g., low flow rate, high flow rate, different flow rate above the low flow rate but below the high flow rate) until the TOC device 10 or another component (e.g., sensor) of the larger system otherwise detects an excursion (e.g., by TOC measurement, by chemical analysis, temperature change, pH level change, other sensor data, etc.) and subsequently activates the different flow rates and related operations unless/until the excursion event is no longer detected.

The differential oxidation exposure approach herein described may reduce sensitivity to various, otherwise error inducing factors including, but not necessarily limited to, flow rate variability, lamp power output, temperature variability, gas bubbles, and background conductivity, combinations thereof, or the like. This approach may provide superior performance on under- or over-reporting at least the following: KHP, SDBS, IPA, MeOH, chloroform, and Urea, by way of non-limiting example. This approach may provide superior accuracy, repeatability, ease of calibrations, and/or improved response time, by way of non-limiting example. This approach may facilitate the generation of full or partial oxidation curves and/or multiple data points for iterative model learning and improvement, identification, and/or classification, by way of non-limiting example.

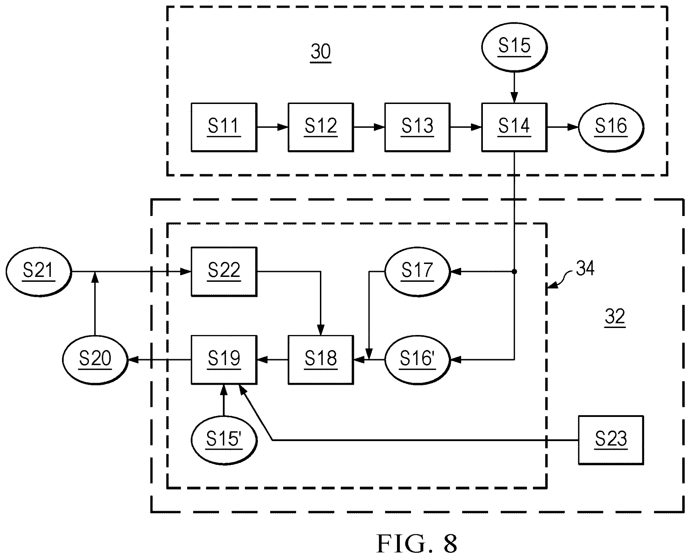

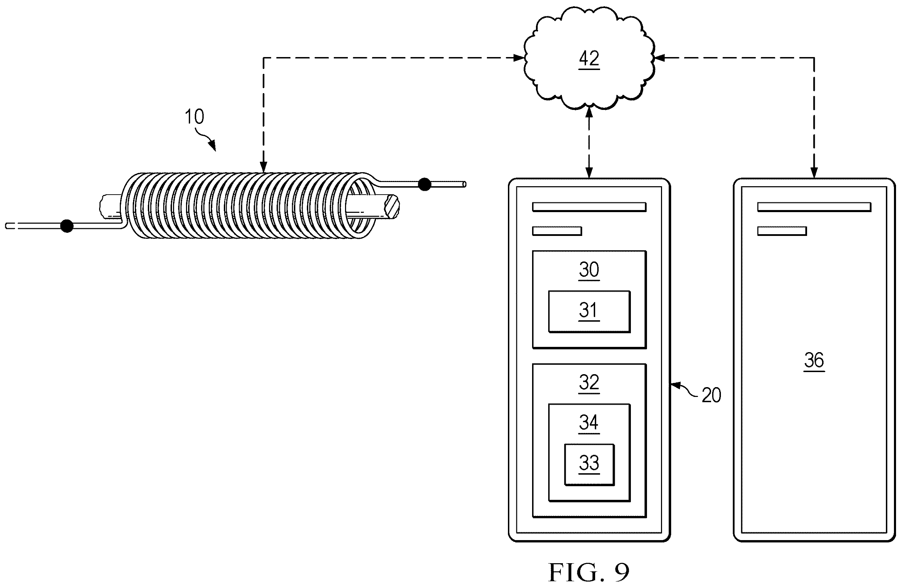

As illustrated with particular regard to **FIGURE 8** and **FIGURE 9,** the multiple data points gathered may be analyzed, such as at the controller 20. Some or all of the controller 20 may be local to the TOC device 10 or remote therefrom and in communication with the TOC device 10 by way of one or more networks 42.

The analysis performed by the controller 20 may be performed by way of a stochastic analysis module 32, which may include an artificial intelligence module 34, and a deterministic analysis module 30 in exemplary embodiments. The deterministic analysis and stochastic analysis modules 30, 32 may be part of the controller 20 and/or TOC device 10, or located separately therefrom and in communication with the controller 20 and/or TOC device 10 by way of one or more networks 42. While used together, in exemplary embodiments, each of the deterministic analysis and stochastic analysis modules 30, 32 may be separately utilized in other exemplary embodiments. Each of the deterministic analysis and stochastic analysis modules 30, 32 may be configured to, in an at least partially automated fashion based on executed software instructions, carry out various steps as shown and/or described herein. The disclosed steps may be performed in different orders, may be omitted, repeated, or the like. While sometimes shown and/or described as separate modules, any number of modules may be utilized, including combining the deterministic analysis and stochastic analysis modules 30, 32 into a common module, or further breaking apart the deterministic analysis and stochastic analysis modules 30, 32 into submodules.

The controller 20 may comprise, or may be in electronic communication with, an expert knowledge system 36 as further described herein.

In exemplary embodiments, without limitation, raw data is acquired at step S11, the data is reduced at step S12, a deterministic model 31, such as of the deterministic analysis module 30, is applied at step S13, and inference with the deterministic model 31 is determined at step S14 to output an initial TOC measurement at step S16, such as in response to a query at step S15 regarding what is the TOC value. Some or all of these steps (S11 through S16) may be performed as part of execution of the deterministic analysis module 30, though such is not required.

In exemplary embodiments, without limitation, following, and optionally in response to step S15, the initial TOC measurement may be provided to the artificial intelligence module 34 of the stochastic analysis module 32 at step S16'. Additionally, an N dimensional tensor may be provided from the deterministic model 31, such as where N >= 0, at step S17, along with the initial TOC measurement of step S16'. A stochastic error model 33 may be applied at step S18. Inference with the stochastic error model 33, application of the expert knowledge system 36, such as by query at step S23, tensor, and initial TOC measurement may be applied at step S19, such as in response to a query regarding what is the TOC error at step S15', to output the error compensated TOC measurement at step S20. The error compensated TOC measurement and/or optionally any user input provided at step S21, may be fed back to the stochastic error model 33, such as by way of a self-learning feedback loop at step S22. Step S23 may optionally occur outside of the artificial intelligence module 34 but as part of the stochastic module 32.

Such analysis may be performed while some or all of the steps of figure 4 are underway or thereafter, by way of non-limiting example. While, at times, a discussion may be made of analyzing multiple data points gathered using the TOC device 10 and/or method of figure 4, the data analysis methods shown and/or described herein may be utilized with multiple data points gathered by other types and/or kinds of TOC sensor devices and/or methods (e.g., batch processing), such as but not necessarily limited to as shown and/or described in US Pat. No. 5,275,957 and/or US Pat. No. 8,618,820, the disclosures of which are hereby incorporated by reference.

As part of executing the deterministic analysis module 30, data, such as raw data, may be acquired at step S11, such as from the sensors 18. The multiple data points may include conductivity measurements from the sensors 18 over time or other sensor data. For example, without limitation, the multiple data points may include conductivity measurements with associated recorded times. The time of the measurement may be recorded by the sensors 18, at the controller 20, by a separate timing device, combinations thereof, or the like.

The data may be reduced at step S12, such as at the controller 20 as part of the deterministic analysis module 30, such as to generate multiple data points. For example, without limitation, such data reduction may include determining a difference in conductivity for sensor 18 measurements, taking an absolute value of the difference, removing noise, and/or eliminating erroneous measurements (e.g., those above/below certain predetermined thresholds, outside a certain number of standard deviations, combinations thereof, or the like), by way of non-limiting example.

The multiple data points may be processed assuming the absence of unpredictability and randomness at step S13, such as part of the deterministic model 31 and/or extrapolation technique. Optionally, an initial TOC measurement may be provided following such processing at step S16. In exemplary embodiments, the deterministic analysis module 30 may use known compensation and/or conversion techniques to convert the delta conductivity measurement to the initial TOC measurement and/or a ppbC measurement, or other measurement. The deterministic model 31 at step S13 may utilize, for example without limitation, certain loss function minimization methods, peak detection techniques, signal integration techniques, combinations thereof, or the like as part of the data analysis. Alternatively, or additionally, the multiple data points may be fit to a curve and/or a curve may be fit to the data, such as using one or more known curve fitting techniques. In exemplary embodiments, without limitation, such curve fitting techniques include a multi-parameter, weighted, regression analysis to essentially fit a curve to the multiple data points, where each parameter represents a quantified state of the oxidation curve. However, other techniques may alternatively or additionally be used such as lookup tables, other curve fitting models, curve identification models, curve extrapolation models, combinations thereof, or the like. Alternatively, or additionally, a regression analysis, extrapolation analysis, or the like may be performed.

Determination and/or provision (e.g., display to an end user) of the initial TOC measurement may occur automatically, and/or in response to a user and/or automated (e.g., by controller 20, with or without manual user input) inquiry at step S15.

As indicated at step S14, in exemplary embodiments, without limitation, a stochastic analysis module 32 may be utilized to help correct for unpredictability and/or randomness caused by the presence/non-presence of various interfering species, temperature changes, UV dosage changes, combinations thereof, or the like, by way of example. At step S16' the initial TOC measurement from the deterministic analysis module 30 may be provided to the stochastic analysis module 32. The stochastic analysis module 32 may allow for learning applications, such as with supervised user input stored at an expert knowledge system 36, though such is not required. The expert knowledge system 36 may comprise, or access, one or more databases which form part of, and/or are accessible by, the stochastic analysis module 32 and/or the deterministic analysis module 30.

The stochastic analysis module 32 may include the artificial intelligence module 34. The multiple data points and/or initial TOC measurement may be processed through the stochastic analysis module 32, including through the artificial intelligence module 34, such as to ultimately arrive at an error compensated TOC measurement (hereinafter also "TOC output") at step S20. As described herein, the error compensated TOC measurement may be a measurement which compensates for multiple or all error parameters.

In exemplary embodiments, without limitation, the artificial intelligence module 34 may be developed by providing a series of training data sets to one or more stochastic error models 33 at step S18, which may comprise one or more neural networks. The training data sets may be manually classified and may represent certain controlled conditions, such as samples with known compositions, at known temperatures, at known flow rates, combinations thereof, or the like. Alternatively, or additionally, the same or different training data sets may be provided to the expert knowledge system 36. The artificial intelligence module 34 may be configured to develop, comprise, and/or utilize a multi-dimensional error analysis hypersurface, such as but not necessarily limited to a three-dimensional error analysis mesh, representing the parameters of the deterministic model 31 and error. The artificial intelligence module 34 may utilize an N dimension tensor, where N >= 0 at step(s) S17, S19, and/or an expert knowledge system 36 at step S19 (optional) to determine an error, such as absolute error, which is applied to the initial TOC measurement at steps S18 and S19 to arrive at the error compensated TOC measurement at step S20. Determination and/or provision (e.g., display to an end user) of the error compensated TOC measurement may occur automatically, and/or in response to a user and/or automated inquiry at step S15'.

The stochastic analysis module 32 may be configured to apply at least certain of the parameters of the deterministic model 31 of step S13 to the multi-dimensional error analysis hypersurface to derive an error for the initial TOC measurement at step(s) S18 and/or S19. The stochastic analysis module 32 may adjust the initial TOC measurement by the derived error to determine the error compensated TOC measurement as provided at step S20. The error compensated TOC measurement may be further compensated as required and provided in and/or converted to ppbC or another measurement. The error compensated TOC measurement may be output and/or converted to various formats (e.g., qualitative or quantitative) using known techniques before final output, though such is not necessarily required.

The expert knowledge system 36 may comprise, or access, one or more databases with one or more rules such as, but not limited to, parameters of the error analysis correlating with certain compounds of interfering species, user specific rules, application specific rules, acceptance or rejection of the oxidation curve data for further artificial intelligence analysis, acceptance or rejection of the regression parameters for further artificial intelligence analysis, rules regarding whether and when to use the artificial intelligence module 34 or another algorithm, using limits to control adjustment to the multi-dimensional error analysis hypersurface (e.g., 3D error analysis mesh) during application of a self-learning stochastic feedback loop at steps S22 and S18, such as with user input as optionally provided at step S21, storing rules for intelligently monitoring and/or controlling a user's water system based on analytic results generated from the artificial intelligence module 34 or another algorithm, combinations thereof, or the like, though any type or kind of rules may be provided. The expert knowledge system 36 can be queried by the artificial intelligence module 34 and/or the deterministic analysis module 30 at any point during the disclosed method, preferably at or between one or more of steps S18-S20.

The stochastic analysis module 32 may utilize the self-learning feedback loop at step S22. In exemplary embodiments, without limitation, the self-learning feedback loop may permit a user to manually adjust error and/or TOC output or otherwise provide other input or rules (e.g., user specific training data sets and/or user specific rules) which are adopted into the artificial intelligence module 34 and/or the expert knowledge system 36, accordingly, such as to provide user specific error compensated measurements. Alternatively, or additionally, the self-learning feedback loop may permit control over various components of the TOC device 10 and/or larger system components (e.g., end user system or components thereof to which the TOC device 10 is connected) based on the findings of the artificial intelligence module 34 (e.g., error compensated TOC measurement or other findings). The information from the self-learning feedback loop may be provided at step S22 to the stochastic error model 33, such as for additional runs of the stochastic analysis module 32 to improve the stochastic analysis at step S19.

While, at times, a discussion may be made of analyzing the multiple data points gathered using the TOC device 10 and/or method of figure 4, the data analysis methods shown and/or described herein may be utilized with other types of sensors, devices, and/or methods (e.g., batch processing, sensors which separate $CO_2$, combinations thereof, of the like). In the case of sensors, devices, and/or methods which separate $CO_2$, the artificial intelligence analysis module 34 may be used to compensate for variability in transfer rate of $CO_2$, environmental conditions, combinations thereof, or the like, by way of example.

In exemplary embodiments, without limitation, the analysis (e.g., generation of oxidation curves, TOC measurements, ppbC measurements, combinations thereof, or the like) are made in real-time. As used in herein, the term real-time may account for normal delays in data transmission and processing, but may exclude deliberate delays of over 1 minute, by way of non-limiting example.

The data analysis methods shown and/or described herein may be performed at the controller 20 or elsewhere (e.g., remote computers, user devices, servers, processors, combinations thereof, or the like). The controller 20 may constitute a single device in a single location, or a single, operational unit which is physically located at disparate locations (e.g., software code and/or other data stored at non-transitory electronic storage devices at more than one location and processed by processors at more than one location).

The results of the analysis, such as in the form of an oxidation curve, TOC measurement(s) (in ppbC or otherwise), multiple data points, insights (e.g., classification results), combinations thereof, or the like, may be visualized at an electronic display 21 local to the TOC device 10, remote therefrom (e.g., as part of a remote user device 23, such as but not necessarily limited to, computer, smartphone, tablet, etc.), combinations thereof, or the like. The results of the analysis may be displayed in real-time, historically, in summary form, combinations thereof, or the like. The results of the analysis may be displayed in quantitative and/or qualitative form, and/or in various formats (numerical measurement, spreadsheet, csv file, graphical plot, etc.). Connection between the controller 20 and the display 21, user device 23, and/or TOC device 10 or components thereof (e.g., sensors 18 and/or flow control device(s) 22) may be made by way of wired and/or wireless connection, such as by way of one or more networks 42, which may comprise the internet, intranet, cellular network, combinations thereof, or the like. Data for and/or from the analysis and/or results of the analysis may be stored at one or more local or remote databases, servers, combinations thereof, or the like. Data for and/or from the analysis and/or results of the analysis may be accessible via website, portal, application, combinations thereof, or the like.

The analysis may be made of various experimental parameters, such as with various temperatures, UV/light dosage, various sample fluids 16, various interfering species, combinations thereof, or the like. Such parameters may be obtained under controlled conditions, such as for use as training data for the artificial intelligence module 34 (e.g., for classification purposes), by way of non-limiting example.

While application to measuring conductivity, such as by way of the sensors 18, is sometimes shown and/or described, other types and kinds of sensors 18 and data measurements may be taken, such as but not limited to: pH, ion selective, NDIR, and other measurement of oxidation products. Data measurements may be derived by converting data from the sensors 18 into various formats, such as converting from uS/cm to ppbC. Additionally known mathematical functions and/or algorithms may be applied to the sensor 18 outputs or applied to data derived from a combination of the sensor 18 outputs during the process of deriving a TOC measurement.

The TOC device 10, such as the controller 20, may be configured to provide alerts, such as electronic notification, upon analysis results (e.g., TOC measurements) meeting certain predetermined rules (e.g., requirements, limits, changes, indications of particular compounds present, combinations thereof, or the like, which may be user defined. The controller 20 may be configured to transmit such alerts to user devices, such as those affiliated with administration of the larger system, and/or to other controllers, such as those affiliated with the larger system, such as to make operational command decisions based on the same in a manual or automated fashion.

Any embodiment of the present invention may include any of the features of the other embodiments of the present invention. The exemplary embodiments herein disclosed are not intended to be exhaustive or to unnecessarily limit the scope of the invention. The exemplary embodiments were chosen and described in order to explain the principles of the present invention so that others skilled in the art may practice the invention. Having shown and described exemplary embodiments of the present invention, those skilled in the art will realize that many variations and modifications may be made to the described invention. Many of those variations and modifications will provide the same result and fall within the spirit of the claimed invention.

Certain operations described herein may be performed by one or more electronic devices. Each electronic device may comprise one or more processors, electronic storage devices, executable software instructions, combinations thereof, and the like configured to perform the operations described herein. The electronic devices may be general purpose computers or specialized computing devices. The electronic devices may comprise personal computers, smartphones, tablets, databases, servers, or the like. The electronic connections and transmissions described herein may be accomplished by one or more wired or wireless connectivity components (e.g., routers, modems, ethernet cables, fiber optic cable, telephone cables, signal repeaters, and the like) and/or networks (e.g., internets, intranets, cellular networks, the world wide web, local area networks, and the like). The computerized hardware, software, components, systems, steps, methods, and/or processes described herein may serve to improve the speed of the computerized hardware, software, systems, steps, methods, and/or processes described herein. The electronic devices, including but not necessarily limited to the electronic storage devices, databases, controllers, or the like, may comprise and/or be configured to hold, solely non-transitory signals.

**Claims**

1. A system for generating multiple data points for total organic carbon ("TOC") analysis of an actively flowing system, said system comprising:

   a TOC device (10) comprising:

   a sample fluid passageway (12);
   a flow control device (22) located at the sample fluid passageway (12);
   one or more conductivity sensors (18) located at the sample fluid (16) passageway (12); and

an oxidation device (14) located at the sample fluid passageway (12); and

a controller (20) comprising one or more non-transitory electronic storage devices comprising software instructions, which when executed, configure one or more processors to:

operate the flow control device (22) to cause a flow of a sample fluid (16) through the sample fluid passageway (12) at a first non-zero flow rate while the oxidation device (14) is active, and take one or more measurements of the sample fluid (16) from the one or more conductivity sensors (18);
operate the flow control device (22) to cause a further flow of the sample fluid (16) through the sample fluid passageway (12) at a second non-zero flow rate while the oxidation device (14) is active, and take one or more further measurements of the sample fluid (16) from the one or more conductivity sensors (18); and
generate a plurality of delta conductivity measurements from the measurements.

2. The system of claim 1, wherein:
the controller (20) comprises additional software instructions stored at the one or more non-transitory electronic storage devices, which when executed, configures the one or more processors to: generate a regression curve from the plurality of delta conductivity measurements.

3. The system of claim 1 or 2, wherein:

the oxidation device (14) comprises an ultraviolet ("UV") light source;
the sample fluid passageway (12) is shaped, at least in part, into a coil about the light source (14); and
the one or more conductivity sensors (18) comprises a first conductivity sensor (18A) positioned at an entrance to the coil and a second conductivity sensor (18B) positioned at an exit of the coil.

4. The system of one of the preceding claims, wherein:

the flow control device (22) comprises at least one of: a pump, a valve, a gas pressure device, and a gravity feed device; and
the first non-zero flow rate is higher than the second non-zero flow rate, wherein preferably the flow of the sample fluid (16) has a first residence time within the sample fluid passageway (12); and wherein preferably the further flow of the sample fluid (16) has a second residence time within the sample fluid passageway (12).

5. The system of one of the preceding claims, wherein:

the one or more measurements comprise a first measurement taken while an entirety of the sample fluid (16) in the sample fluid passageway (12) is at the first non-zero flow rate; and
the one or more further measurements comprise:

a first further measurement taken while the sample fluid (16) in the sample fluid passageway (12) comprises a first portion at the first non-zero flow rate and a second portion at the second non-zero flow rate; and
a second further measurement taken while an entirety of the sample fluid (16) in the sample fluid passageway (12) is, and has only been, at the second non-zero flow rate.

6. The system of one of the preceding claims, wherein:
the controller (20) comprises additional software instructions stored at the one or more non-transitory electronic storage devices, which when executed, configures the one or more processors to: cycle between at least the first non-zero flow rate and the second non-zero flow rate, and wherein preferably the controller comprises additional software instructions stored at the one or more non-transitory electronic storage devices, which when executed, configures the one or more processors to: continuously cycle between the first non-zero flow rate and the second non-zero flow rate in the bi-modal fashion

7. The system of one of the preceding claims, wherein:
the controller (20) is remote from the TOC device.

8. The system of one of the preceding claims, wherein:
the controller (20) comprises additional software instructions stored at the one or more non-transitory electronic storage devices, which when executed, configures the one or more processors to: determine a ppbC measurement from the plurality of delta conductivity measurements.

9. The system of one of the preceding claims, wherein:
the controller (20) comprises additional software instructions stored at the one or more non-transitory electronic storage devices, which when executed, configures the one or more processors to: apply a deterministic model to the plurality of delta conductivity measurements to arrive at an initial TOC measurement, and wherein preferably the controller comprises additional software instructions stored at the one or more non-transitory electronic storage devices, which when executed, configures the one or more processors to: apply an artificial intelligence module comprising a stochastic error model to the initial TOC measurement based on the plurality of delta conductivity measurements to arrive at an error compensated TOC measurement.

10. A method for generating multiple data points for total organic carbon ("TOC") analysis of an actively flowing system, said method comprising:

operating a flow control device (22) to cause a cyclic flow of a sample fluid (16) through a sample fluid passageway (12) between a plurality of non-zero flow rates while an oxidation device (14) proximate the sample fluid passageway (12) is active, and taking one or more measurements of the sample fluid (16) from one or more conductivity sensors (18) located at the sample fluid passageway (12) at different times during the cycle; and generating a plurality of delta conductivity measurements from the measurements.

11. The method of claim 10, further comprising:
fitting, at a controller (20), a curve to the plurality of delta conductivity measurements.

12. The method of claim 10 or 11, wherein:

the sample fluid passageway (12) is shaped, at least in part, into a coil about a light source (14);
the light source (14) comprises an ultraviolet ("UV") lamp;
the one or more conductivity sensors (18) comprises a first conductivity sensor (18A) positioned at an entrance to the coil and a second conductivity sensor (18B) positioned at an exit of the coil; and
the flow control device (22) comprises at least one of: a pump, a valve, a gas pressure device, and a gravity feed device.

13. The method of one of the claims 10 to 12, wherein:

the one or more measurements comprise a first measurement taken while an entirety of the sample fluid (16) in the sample fluid passageway (12) is at the first flow rate; and
the one or more additional measurements comprise:

a first additional measurement taken while the sample fluid (16) in the sample fluid passageway (12) comprises a first portion of the flow at the first flow rate and a second portion of the flow at the second flow rate; and
a second additional measurement taken while an entirety of the sample fluid (16) in the sample fluid passageway (12) is, and has been only at, the second flow rate.

14. The method of one of the claims 10 to 13, further comprising:
applying, at the controller (20), a deterministic model to the plurality of delta conductivity measurements to arrive at an initial TOC measurement, wherein preferably the method further comprises: applying, at the controller (20), an artificial intelligence module (34) comprising a stochastic error model (33) to the initial TOC measurement based on the plurality of delta conductivity measurements to arrive at an error compensated TOC measurement, and wherein preferably the method further comprises: determining, at the controller (20), a ppbC measurement from the error compensated TOC measurement.

15. A system for generating multiple data points for total organic carbon ("TOC") analysis of an actively flowing system, said system comprising:

a TOC device (10) comprising:

a sample fluid passageway (12) for a sample fluid (16) comprising a coiled portion;

a flow control device (22) located at an entrance or an exit to the coiled portion of the sample fluid passageway (12), said flow control device (22) comprising at least one of: a pump, a valve, a gas pressure device, and a gravity feed device;

a first conductivity sensor (18A) located at the entrance to the coiled portion of the sample fluid passageway (12);

a second conductivity sensor (18B) located at the exit to the coiled portion of the sample fluid passageway (12); and

an ultraviolet ("UV") lamp (14) located within the coiled portion of the sample fluid passageway (12) for irradiating the sample fluid (16) within at least the coiled portion of the sample fluid passageway (12), when activated; and

a controller (20) comprising one or more non-transitory electronic storage devices comprising software instructions, which when executed, configure one or more processors to:

operate the flow control device (22) to cause a flow of the sample fluid (16) through the sample fluid passageway (12) at a first flow rate while the UV lamp (14) is active and irradiating the flow of the sample fluid (16);

take a first conductivity measurement of the sample fluid (16) from the first conductivity sensor (18A) and the second conductivity sensor (18B) while the coiled portion of the sample fluid passageway (12) contains only the flow of the sample fluid (16) at the first flow rate;

operate the flow control device (22) to cause a further flow of the sample fluid (16) through the sample fluid passageway (12) at a second flow rate while the UV lamp (14) is active and irradiating the further flow of the sample fluid (16), where the second flow rate is lower than the first flow rate;

take a second conductivity measurement of the sample fluid (16) from the first conductivity sensor (18A) and the second conductivity sensor (18B) while the coiled portion of the sample fluid passageway (12) contains the flow of the sample fluid (16) at the first flow rate and at the second flow rate;

take a third conductivity measurement of the sample fluid (16) from the first conductivity sensor (18A) and the second conductivity sensor (18B) while the coiled portion of the sample fluid passageway (12) contains only the flow of the sample fluid (16) at the second flow rate; and

generate a plurality of delta conductivity measurements from the first, second, and third conductivity measurements.

FIG. 1

FIG. 2

DELTA CONDUCTIVITY

RESIDENCE TIME

+/- MEASUREMENT
ERROR

24

FIG. 3

DELTA CONDUCTIVITY

RESIDENCE TIME

24

FIG. 4

FIG. 5

FIG. 6A

FIG. 6B

FIG. 6C

FIG. 7A

FIG. 7B

FIG. 8

FIG. 9

Europäisches
Patentamt
European
Patent Office
Office européen
des brevets

## EUROPEAN SEARCH REPORT

**Application Number**

EP 25 20 5546

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 798 271 A (GODEC RICHARD D [US] ET AL) 25 August 1998 (1998-08-25) | 1,3-15 | INV.<br>G01N33/18<br>G01N27/08 |
| Y | * column 1, line 19 - column 8, line 21 *<br>* column 9, line 13 - column 12, line 33; figures *<br>* column 14, line 21 - line 67 *<br>----- | 2 | |
| Y | WO 2004/027387 A2 (METTES JACOB [US])<br>1 April 2004 (2004-04-01) | 2 | |
| A | * page 2, paragraph 2 *<br>* page 4 - page 7 *<br>----- | 1,3-15 | |
| A | US 2010/204926 A1 (METTES JACOB [US])<br>12 August 2010 (2010-08-12)<br>* paragraph [0010] - paragraph [0027]; figures *<br>----- | 1-15 | |

**TECHNICAL FIELDS SEARCHED (IPC)**

G01N

The present search report has been drawn up for all claims

| Place of search | Date of completion of the search | Examiner |
|---|---|---|
| The Hague | 6 February 2026 | Savage, John |

EPO FORM 1503 03.82 (P04C01)

## ANNEX TO THE EUROPEAN SEARCH REPORT
## ON EUROPEAN PATENT APPLICATION NO.

EP 25 20 5546

This annex lists the patent family members relating to the patent documents cited in the above-mentioned European search report.
The members are as contained in the European Patent Office EDP file on
The European Patent Office is in no way liable for these particulars which are merely given for the purpose of information.

06-02-2026

| Patent document cited in search report | | Publication date | Patent family member(s) | | Publication date |
|---|---|---|---|---|---|
| US 5798271 | A | 25-08-1998 | EP | 0897538 A1 | 24-02-1999 |
| | | | US | 5798271 A | 25-08-1998 |
| | | | WO | 9738304 A1 | 16-10-1997 |
| WO 2004027387 | A2 | 01-04-2004 | AU | 2003276917 A1 | 08-04-2004 |
| | | | EP | 1581813 A2 | 05-10-2005 |
| | | | WO | 2004027387 A2 | 01-04-2004 |
| US 2010204926 | A1 | 12-08-2010 | US | 2005165575 A1 | 28-07-2005 |
| | | | US | 2010204926 A1 | 12-08-2010 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5132094 A
- US 9791430 B
- US 5275957 A
- US 8618820 B